# FASCICULE DE BREVET EUROPEEN

(11) **EP 0 670 715 B1**
(45) Date de publication et mention de la délivrance du brevet: **07.05.1997**
(21) Numéro de dépôt: 94928928.4
(22) Date de dépôt: 29.09.1994
(51) Int. Cl.: A61K 9/107

(54) **COMPOSITION ADMINISTRABLE PAR VOIE ORALE APTE A FORMER UNE MICROEMULSION**
ZUSAMMENSETZUNG, DIE BEI PERORALER VERABREICHUNG EINE MIKROEMULSION BILDET
ORAL DELIVERY COMPOSITION FORMING A MICROEMULSION

(30) Priorité: 30.09.1993 FR 9311876
(43) Date de publication de la demande: 13.09.1995
(73) Titulaire: GATTEFOSSE S.A., 69800 Saint Priest (FR)
(72) Inventeur: FARAH, Nabil, F-69007 Lyon (FR); DENIS, Joel, F-69390 Charly (FR)
(74) Mandataire: Vuillermoz, Bruno
(86) Numéro de dépôt international: FR9401137
(87) Numéro de publication internationale: WO9508983

(56) Documents cités:
- EP-A- 0 152 945
- WO-A-93/23083

## Description

### Domaine technique

L'invention concerne une composition administrable notamment par voie orale, à usage pharmaceutique ou cosmétique, apte à former insitu une micro-émulsion avec le liquide biologique de l'organisme ; l'invention concerne plus particulièrement une composition formée d'un système vecteur d'actifs auto-micro-émulsionnable, dénommé dans le métier par l'expression anglaise "SMEDDS" (self micro emulsifying drug delivery system) ; ces systèmes présentent la propriété de s'émulsionner dans l'eau à la température du corps humain.

Cette composition est destinée d'une part à véhiculer un ou plusieurs actifs, solubles ou peu solubles, et d'autre part à former avec le liquide biologique de l'organisme humain une micro-émulsion, sachant qu'un ou des actifs en solution dans une micro-émulsion présente une meilleure biodisponibilité.

### Techniques antérieures

Comme on le sait, une micro-émulsion est une solution homogène, fluide et stable, composée de quatre constituants majeurs respectivement une phase hydrophile, une phase lipophile, au moins un agent tensioactif (TA) et au moins un agent cotensiactif (CoTA).

Les micro-émulsions ont été largement étudiées pour la récupération du pétrole. Il n'y a donc pas lieu de les décrire ici en détail.

Un agent tensioactif est un composé chimique possédant deux groupements, le premier polaire ou ionique, qui présente une grande affinité pour l'eau, le second qui contient une chaîne aliphatique plus ou moins longue et est hydrophobe ; ces composés chimiques à caractère hydrophile prononcé, sont destinés à provoquer la formation de micelles en solution aqueuse ou huileuse.

Un agent cotensioactif dénommé également parfois "agent cosurfactif", est également un composé chimique, mais à caractère hydrophobe, destiné à provoquer la solubilisation mutuelle des phases aqueuses et huileuses dans une micro-émulsion.

L'invention vise donc à augmenter la biodisponibilité des principes actifs difficilement solubles, par leur solubilisation au sein d'une micro-émulsion.

Dans le document EP-A-0 334777, le Demandeur a montré la possibilité d'utiliser des micro-émulsions dans l'industrie pharmaceutique. Dans le document WO 93/12766, le Demandeur a décrit des compositions particulières à usage pharmaceutique, sous forme de micro-émulsions destinées à être conditionnées sous forme de suppositoires, dans lesquelles la phase hydrophile est apportée par le liquide rectal. Cette réalisation donne d'excellents résultats pour des suppositoires, mais ne peut convenir pour des compositions destinées à être ingérées per-os, car la quantité de liquide biologique dans l'estomac ou l'intestin est beaucoup plus importante que dans le rectum (plusieurs décilitres). Or les conditions d'existence des micro-émulsions telles que celles décrites dans le document WO 93/12766 nécessitent un très faible pourcentage d'eau (quelques millilitres).

Dans le document EP-A-0152945, on a décrit un système du type en question, constitué d'une phase lipophile formée d'alcool oléïque et/ou d'esters polaires d'alcools en C2-C4 avec des acides gras en C8-C12, et en tant qu'agent tensio-actif, de la glycérine éthoxylée, le mélange contenant de 35 à 85% en poids d'eau. Ces formulations contiennent de l'eau en proportion appréciable, ce qui exclut la forme de présentation en gélules. En revanche, les compositions contenant de l'eau forment une macro-émulsion in situ et non pas une micro-émulsion, ce qui diminue fortement la biodidponibilité.

Dans le document WO 93/23083, publié après la date de priorité de la présente demande, on a décrit un système dans lequel la phase hydrophile anhydre est constituée par un glycéride polyglycolisé à chaînes courtes en C8-C10. Ce système n'améliore pas de façon significative la diffusion membranaire des principes actifs. Il ne permet pas de véhiculer des principes actifs hydrophiles et n'autorise pas la formation d'émulsion, et a fortiori de micro-émulsion in situ, ce qui ne facilite pas la biodisponibilité.

Les industries pharmaceutique et cosmétique demandent de plus en plus des compositions exemptes de phase aqueuse, afin de faciliter leur conditionnement, sous forme de gélules, de comprimés, d'emplâtres. Les compositions connues à ce jour pour la fabrication des gélules, notamment celles décrites dans les documents ci-dessus, ne peuvent convenir, car la présence de l'eau contenue dans ces mélanges est incompatible avec la technique de la gélule.

L'invention résout ces problèmes. Elle vise une composition administrable par voie orale, notamment à usage pharmaceutique ou cosmétique comprenant une phase lipophile, au moins un agent tensioactif, et au moins un agent cotensioactif qui en mélange et en présence du liquide physiologique, forment une micro-émulsion facilitant la dissolution in situ et améliorant la biodisponibilité des principes actifs.

### Exposé de l'invention

Cette composition administrable par voie orale, apte à former une micro-émulsion, comprenant au moins :
. un actif,
. une phase lipophile constituée par un mélange de glycérides et d'esters d'acides gras,
. un agent tensioactif (TA),
. un agent cotensioactif (CoTA),
. une phase hydrophile,
se caractérise :
- en ce que la phase lipophile est constituée par un mélange de glycérides polyglycolysés en C₈ à C₁₈, présentant une balance hydrophile-lipophile (HLB) inférieure à 16, cette phase lipophile représentant de 1 à 75 % du poids total de la composition;
- en ce que l'agent tensioactif (TA) est choisi dans le groupe comprenant les glycérides polyglycolysés saturés en C₈-C₁₀ et les esters oléiques du polyglycérol, cet agent tensioactif présentant également une HLB inférieure à 16 ;
- en ce que l'agent cotensioactif (CoTA) est choisi dans le groupe comprenant les esters lauriques du propylène glycol, les esters oléiques du polyglycérol et l'éthyl diglycol ;
- en ce que le rapport TA/CoTA est compris entre 0,5 et 6 ;
- et en ce que la phase hydrophile de la micro-émulsion finale est apportée après ingestion par le liquide physiologique du milieu digestif.

En d'autres termes, l'invention consiste à avoir sélectionné une phase lipophile particulière qui, combinée avec des agents tensioactifs et des agents cotensioactifs spécifiques, forme une micro-émulsion en présence du liquide physiologique de l'estomac et de l'intestin de l'organisme humain ou animal. De la sorte, il n'est pas nécessaire d'apporter une phase hydrophile extérieure pour réaliser cette micro-émulsion. Cette composition améliore la biodisponibilité des principes actifs au sein de l'organisme. La composition selon l'invention peut ainsi être efficacement administrée par voie orale.

De manière surprenante, l'utilisation de phase lipophile à longueur de chaînes importante permet d'améliorer de façon significative la diffusion membranaire et le passage des principes actifs dans le sang.

Dans la description et dans les revendications:
- par "glycérides polyglycolysés", on désigne un mélange de mono-, di- et triglycérides et de mono- et di-esters de polyéthylène-glycol (PEG), de poids moléculaire compris de préférence entre 200 et 600, éventuellement de glycérol et de PEG libres, dont on règle la valeur HLB par la longueur de la chaine du PEG et dont on règle le point de fusion par la longueur des chaines des acides gras, du PEG et du degré de saturation des chaînes grasses, donc de l'huile de départ;
- par "acide gras en C₈ à C₁₈" que l'on écrit aussi C₈-C₁₈, on désigne des mélanges, en proportions significatives et variables des acides caprylique (C₈), caprique (C₁₀), laurique (C₁₂), myristique (C₁₄), palmitique (C₁₆) et stéarique (C₁₈), lorsque ces acides sont saturés et des acides insaturés correspondants en C₈-C₁₈. Les proportions de ces acides gras peuvent varier en fonction des huiles de départ.

Avantageusement, en pratique :
- les glycérides polyglycolysés de la phase lipophile sont saturés, ce qui permet de réaliser des mélanges solides à température ambiante et liquide à température du corps humain ; ce mélange présente une HLB inférieure 16, de préférence comprise entre 9 et 15, avantageusement voisine de 14, du type de celui commercialisé par le Demandeur sous la marque déposée "GELUCIRE 44/14" ; en effet, si la valeur HLB est supérieure à 16, le mélange devient trop hydrophile, ce qui ne favorise pas la micro-émulsion. On a observé que l'on obtient les meilleurs résultats lorsque la valeur de HLB voisine de 14 car on peut former des micro-émulsions dans une zone la plus large.
- dans une variante, on utilise un mélange de glycérides polyglycolysés insaturés en C₈-C₁₈, de HLB égale à 6 et liquide à température ambiante, tel que celui commercialisé par le Demandeur sous la marque déposée "LABRAFIL WL 2609 BS" ;
- la phase lipophile représente de 1 à 75 % du poids de la composition et de préférence entre 10 à 75 % du poids de la composition ; on a observé qu'hors de ces limites, on ne peut former de micro-émulsion largement diluable ;
- l'agent tensioactif (TA) est formé de glycérides polyglycolysés en C₈-C₁₀, de HLB inférieure à 16, de préférence comprise entre 5 et 14, tel que ceux commercialisés par le Demandeur sous les marques déposées "LABRAFAC CM 10", "LABRAFAC HYDROPHILE" ou "LABRASOL" ;
- dans une variante, l'agent tensioactif (TA) est constitué par des esters oléiques du polyglycérol, de HLB égale à 10, du type de celui commercialisé par le Demandeur sous la marque déposée "PLUROL OLEIQUE" ;
- l'agent cotensioactif (CoTA) est un éthyl diglycol (encore dénommé éther monoéthylique du diéthylène glycol), tel que celui commercialisé par le Demandeur sous la marque déposée "TRANSCUTOL";
- dans une variante, l'agent cotensioactif (CoTA) est constitué d'esters lauriques de propylène glycol du type de celui commercialisé par le Demandeur sous la marque déposée "LAUROGLYCOL";
- dans une autre variante, l'agent cotensioactif (CoTA) est constitué par des esters oléiques du polyglycérol, du type de celui commercialisé par le Demandeur sous la marque déposée "PLUROL OLEIQUE" ;
- le rapport TA/CoTA est compris entre 0,5 et 6, de préférence entre 1 et 2. Au dessus de 6 et en dessous de 0,5, la composition ne donne pas de micro-émulsion fortement diluable.

Un des principaux intérêts de l'invention est que, quelle que soit la quantité d'eau apportée par le liquide physiologique gastrique ou intestinal de l'organisme humain ou animal (de l'ordre de quelques décilitres), le mélange formé de cette quantité d'eau et de la composition formera une micro-émulsion, favorable à la solubilité du principe actif, qui augmente la biodisponibilité et ce malgré la proportion appréciable de ce liquide physiologique.

Il a donc fallu déterminer les zones d'existence des micro-émulsions en question. Pour cela, on a tracé des diagrammes pseudo-ternaires, en conservant un rapport tensioactif TA/cotensioactif CoTA de 1. On a réalisé des mélanges phase lipophile + TA + CoTA en différentes proportions. Sur chacun de ces mélanges, on a ensuite ajouté goutte à goutte la phase hydrophile jusqu'à obtention d'une solution limpide. Cette quantité ajoutée correspond au pourcentage minimum de la phase hydrophile nécessaire pour entrer dans la zone micro-émulsionnée. C'est le pourcentage dit d'entrée. On continue alors à ajouter la phase hydrophile, jusqu'à l'apparition d'un trouble. La quantité ajoutée correspond alors au pourcentage dit de sortie.

Cette méthode de construction des diagrammes pseudo-ternaires est appelée "méthode de titration". Le domaine d'existence de la micro-émulsion, solution claire et limpide, est compris entre deux domaines d'existence d'une émulsion, dispersion à l'aspect laiteux.

Si le plus généralement la coposition initiale destinée à être ingérée ne contient pas d'eau, il est utile toutefois pour certaines utilisations que cette composition contienne de l'eau sous reserve néanmoins que celle-ci ne participe pas initialement à la formation de la micro-émulsion finale.

En effet, lorsque la composition se présente sous forme liquide, donc hygroscopique par nature, et destinée à être conditionnée sous forme de gélules, la présence d'eau en faible proportion peut être utile pour équilibrer la reprise d'eau contenue dans la tunique d'encapsulage. On conçoit aisément que cette eau ne participe pas à la formation de la micro-émulsion finale, mais tout simplement permet la stabilité de la gélule et évité qu'elle ne devienne cassante.

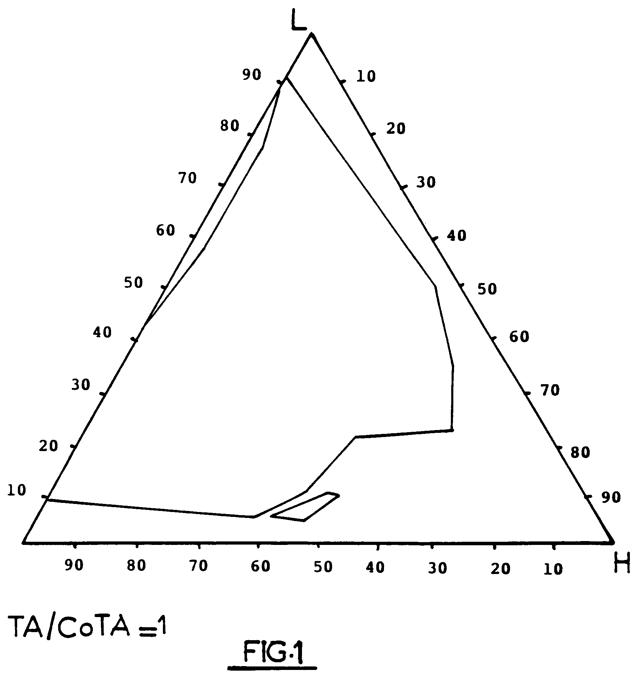

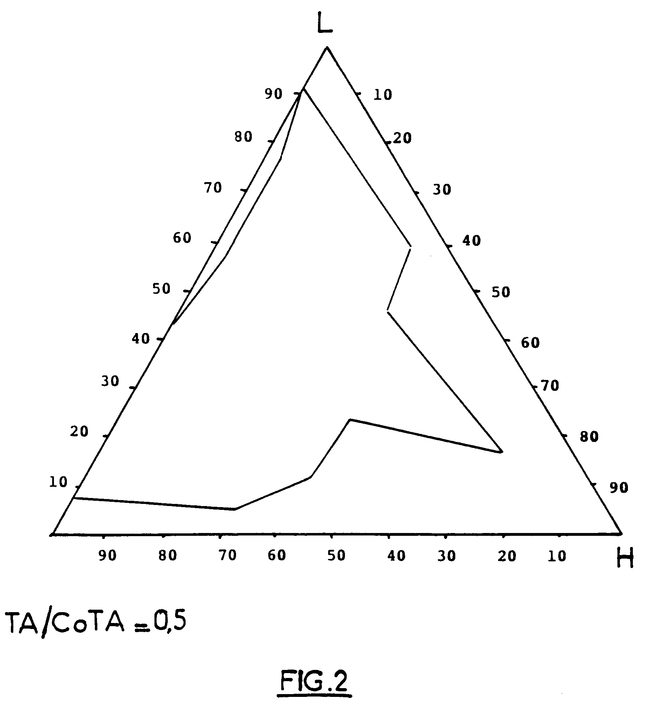

Les figures 1 et 2 représentent les diagrammes pseudo-ternaires de deux compositions préférées de l'invention.

Dans les deux cas, le mélange tensioactif (TA)-cotensioactif (CoTA) est le même. Seule la phase lipophile diffère. Sur les deux diagrammes, il apparaît clairement que la zone d'existence de la micro-émulsion est très large et correspond à un pourcentage de phase hydrophile important (de 0 à 60 % sur la figure 1, de 0 à 70 % sur la figure 2).

Par conséquent, la micro-émulsion pourra se former, même au sein de l'appareil digestif, où la quantité de liquide biologique est importante (de l'ordre de quelques décilitres).

Les exemples qui suivent permettent de visualiser des compositions préférées de l'invention. Il va de soi que le pourcentage en principes actifs est fonction de la posologie du principe actif administré.

Dans tous les exemples, le complément en poids à 100 % de la composition est précisément représenté par un principe actif anti-inflammatoire, à savoir l'indométacine dans les exemples 1 à 8, 11 et 12, l'hydrocortisone à l'exemple 9 et le diclofénac de sodium à l'exemple 10.

### Manières de réaliser l'invention

### Exemple 1 :

Comme phase lipophile, on utilise un mélange de glycérides polyglycolysés saturés en C₈-C₁₈ présentant un point de fusion de 48°C et une HLB de 9, commercialisé par le Demandeur sous la dénomination "GELUCIRE 48/09". Cette phase représente 73,7 % du poids total de la composition finale.

Le mélange tensioactif (TA)-cotensioactif (CoTA) est constitué d'un mélange de glycérides polyglycolysés en C8-C10 (TA), de HLB égale à 10, commercialisé par le Demandeur sous la dénomination "LABRAFAC CM 10", et d'esters lauriques de propylène glycol (CoTA) du type de celui commercialisé par le Demandeur sous la dénomination "LAUROGLYCOL". Ce mélange représente 18,5 % du poids de la composition et le rapport TA/CoTA est égal à 0,5.

La composition obtenue est solide à température ambiante, et devient liquide à 37°C. Elle présente de nombreux avantages par rapport aux formulations classiques utilisées avec le même principe actif. On peut noter un excellent pouvoir de solubilisation du principe actif dans le milieu digestif, une bonne biodisponibilité, bien que ce principe actif soit lipophile.

Les tests de dissolution in vitro dans un milieu gastrique à pH 1,2, ont montré qu'avec cette formulation, on arrive à un pourcentage de 48 % du principe actif dissous au bout d'une heure. A titre de comparaison, lorsque l'on teste le principe actif pur (indométacine), en une heure, on ne dissout au mieux que 5 % de ce principe actif ; et toujours à titre de comparaison, avec des gélules de ce principe actif actuellement commercialisées, on obtient au bout d'une heure seulement 4,7 % de principe actif dissous.

On ne pouvait pas prévoir que la composition selon l'invention permette d'améliorer d'autant (près de dix fois) la dissolution des principes actifs.

### Exemple 2 :

Comme phase lipophile, on utilise un mélange de glycérides polyglycolysés saturés en C₈-C₁₈ présentant un point de fusion de 44°C et une HLB de 14, commercialisé par le Demandeur sous la dénomination "GELUCIRE 44/14" : ce mélange représente 52,5 % du poids total de la composition finale.

Pour le mélange tensio actif + cotensioactif, on utilise les mêmes composants qu'à l'exemple 1 : ce mélange TA+CoTA représente cette fois 35 % du poids de la composition, et le rapport TA/CoTA est égal à 0,5.

On observe les mêmes propriétés qu'à l'exemple 1.

### Exemple 3 :

On utilise la même phase lipophile et en même proportion qu'à l'exemple 2.

Le mélange tensioactif + cotensioactif est constitué du même tensioactif qu'aux exemples 1 et 2 associé à un cotensioactif constitué par l'éthyl diglycol, commercialisé par le Demandeur sous la dénomination "TRANSCUTOL"; ce mélange TA+CoTA représente 35 % du poids total de la composition finale et le rapport TA/CoTA est égal à 0,5.

### Exemple 4 :

On utilise la même phase lipophile qu'à l'exemple 2. Cette phase lipophile représente 73,7 % du poids total de la composition finale.

On utilise le même mélange TA+CoTA qu'à l'exemple 2 ; ce mélange représente 18,5 % du poids de la composition finale, le rapport TA/CoTA est égal à 0,5.

### Exemple 5 :

On utilise la même phase lipophile et en même proportion qu'à l'exemple 2.

Le mélange tensioactif + cotensioactif est constitué d'un mélange d'esters oléiques du polyglycérol, de HLB égale à 10, commercialisé par le Demandeur sous la dénomination "PLUROL OLEIQUE" pour le tensioactif, l'agent cotensioactif étant le même qu'à l'exemple 3, ce mélange représentant 35 % du poids de la composition, le rapport TA/CoTA étant égal à 1.

Cet exemple est illustré par le diagramme pseudo-ternaire de la figure 1, dans lequel la référence L désigne la phase lipophile et la référence H la phase hydrophile. La zone d'existence de la micro-émulsion est très large et correspond à un pourcentage d'eau élevé (entre 0 et 60 %). De la sorte, la composition contenant les actifs est largement diluable, ce qui lui confère un bon pouvoir solubilisant dans le système digestif et une bonne biodisponibilité des actifs, ce que l'on ne savait pas obtenir à ce jour.

### Exemple 6 :

Comme phase lipophile, on utilise un mélange de glycérides polyglycolysés saturés en C₈-C₁₈ présentant un point de fusion de 42°C et une HLB de 12, commercialisé par le Demandeur sous la dénomination "GELUCIRE 42/12".

On utilise le même couple tensioactif + cotensioactif qu'à l'exemple 1, et on remplace la phase lipophile par un mélange de glycérides polyglycolysés saturés en C₈-C₁₈ présentant un point de fusion de 42°C et une HLB de 12.

Cet exemple est illustré par le diagramme pseudo-ternaire de la figure 2. La zone d'existence de la micro-émulsion est très large et correspond à un pourcentage d'eau élevé (entre 0 et 70 %).

### Exemple 7 :

On utilise comme phase lipophile un mélange de glycérides polyglycolysés saturés en C8-C18 présentant un point de fusion de 44°C et une HLB de 14 commercialisée par le Demandeur sous la dénomination "GELUCIRE 44/14". Cette phase représente 45,2 % du poids total de la composition finale.

Comme agent tensio actif TA, on utilise le même mélange qu'à l'exemple 1, et ce en proportion de 3,7 % du poids total de la composition.

Comme agent cotensioactif, on utilise le même composé qu'à l'exemple 5, dénommé "PLUROL OLEIQUE", cette fois agissant en tant qu'agent cotensioactif, et ce à raison de 7,5 %.

Le rapport TA/CoTA est égal à 1 et le principe actif (indométacine) représente 43,5 % du total.

### Exemple 8:

Des tests in vivo ont été conduits sur des rats avec comme principe actif l'indométacine.

Les proportions en poids des différents constituants de ce mélange sont:

| | | |
|---|---|---|
| Tensio-actif: | LABRASOL | 44,90% |
| Cotensio- actif: | PLUROLOLEIQUE | 15,00% |
| Phase lipophile: | LABRAFIL WL 2609 BS | 39,90% |
| Principe actif: | Indométacine | 0,20% |

On procède premièrement à une administration orale de l'Indométacine selon la composition ci-dessus. Les résultats montrent qu'après une heure, les concentrations plasmatiques se situent entre 5 et 7 microgrammes par millilitre (µg/ml), et après deux heures entre 7 et 13 µg/ml.

Une deuxième série de tests consiste en une administration orale d'indométacine sous forme de poudre mise en suspension extemporanée. On constate qu'après une heure, les concentrations plasmatiques se situent entre 1 et 5 µg/ml et après deux heures, entre 1 et 4 µg/ml. La cinétique obtenue est irrégulière et varie d'un animal à l'autre.

La biosdisponibilité est environ deux fois plus grande dans le cas de l'administration selon l'invention.

### Exemple 9:

Dans cet exemple on compare les cinétiques en milieu gastrique (à pH 1,2) de la dissolution de l'hydrocortisone.

On utilise la même phase lipophile et le même mélange TA/CoTA qu'à l'exemple 8 mais dans les proportions suivantes:
- - Hydrocortisone: 4,0%
- - Phase lipophile: 19,2%
- - Tensioactif: 57,6%
- - Co-tensioactif: 19,2%

On peut ainsi obtenir une cinétique de dissolution in vitro allant jusqu'à 50% en 45 minutes (mn) alors que l'hydrocortisone sous forme de poudre présente une dissolution de 10% en 45 mn.

### Exemple 10:

Dans cet exemple on compare les cinétiques de dissolution in vitro en milieu gastrique (à pH 1,2) dans lequel le diclofénac de sodium sous forme de poudre est pratiquement insoluble.

Les proportions en poids des différents constituants de ce mélange sont:

| | | |
|---|---|---|
| Tensio-actif: | LABRASOL | 38,40% |
| Cotensio- actif: | PLUROLOLEIQUE | 38,40% |
| Phase lipophile: | LABRAFIL WL 2609 BS | 19,20% |
| Principe actif: | diclofénac de sodium | 4,00% |

On peut ainsi obtenir une cinétique de dissolution allant jusqu'à 86% en 30 mn alors que le diclofénac de sodium sous forme de poudre présente une cinétique de dissolution de 1% dans le même temps.

### Exemple 11:

Dans cet exemple, on compare les cinétiques de dissolution in vitro en milieu gastrique (à pH=1,2) dans lequel l'indométacine seule a une cinétique de dissolution inférieure à 5% dans le même milieu.

Les proportions en poids des différents constituants de ce mélange sont:

| | | |
|---|---|---|
| Tensio-actif: | LABRASOL | 57,60% |
| Cotensio- actif: | PLUROLOLEIQUE | 19,20% |
| Phase lipophile: | LABRAFIL WL 2609 BS | 19,20% |
| Principe actif: | Indométacine | 4,00% |

On obtient une cinétique de dissolution de 57,1% en 30 mn.

### Exemple 12:

Dans cet exemple, on utilise les mêmes composants que dans l'exemple précédent avec proportions différentes, à savoir:

| | | |
|---|---|---|
| Tensio-actif: | LABRASOL | 43,40% |
| Cotensio- actif: | PLUROLOLEIQUE | 14,40% |
| Phase lipophile: | LABRAFIL WL 2609 BS | 38,40% |
| Principe actif: | Indométacine | 4,00% |

On obtient une cinétique de dissolution de 90% en 30 mn. On observe qu'une augmentation de la phase lipophile améliore la dissolution dans des proportions significatives.

Les compositions conformes à l'invention présentent de nombreux avantages par rapport à celles déjà connues. On peut citer l'absence de phase aqueuse, qui facilite le conditionnement de ces compositions pharmaceutiques, en particulier sous forme de gélules, de capsules molles, de comprimés ou d'emplâtres.

L'invention peut donc ainsi être administrée par voie orale.

Il s'avère donc que l'invention permet d'élargir le choix à la fois du conditionnement et de l'administration de compositions à usage pharmaceutique ou cosmétique. En conséquence, l'exploitation industrielle des compositions conformes à l'invention sera facilitée et pourra être envisagée avec succès.

## Revendications

1. Composition à usage pharmaceutique destinée à être ingérée, apte à former une microémulsion, comprenant au moins :
. un actif,
. une phase lipophile représentant de 1 à 75 % du poids total de la composition, constituée par un mélange de glycérides et d'esters d'acides gras en C₈ à C₁₈,
. un agent tensioactif (TA) à base de glycérides présentant une balance hydrophile-lipophile (HLB) inférieure à 16,
. un agent cotensioactif (CoTA) formé par un ester d'alcools polyvalents, le rapport TA/CoTA est compris entre 0,5 et 6,
. une phase hydrophile,
caractérisée :
- en ce que les glycérides de la phase lipophile sont constitués par un mélange de mono-, di-, et triglycérides et de mono- et diesters de polyéthylène glycol, présentant une balance hydrophile-lipophile (HLB) inférieure à 16 ;
- en ce que l'agent tensioactif (TA) est choisi dans le groupe comprenant les glycérides polyglycolysés saturés en C₈-C₁₀ et les esters oléïques du polyglycérol ;
- en ce que l'agent cotensioactif (CoTA) est choisi dans le groupe comprenant les esters lauriques du propylène glycol, les esters oléïques du polyglycérol et l'éthyl diglycol ;
- et en ce que la phase hydrophile de la microémulsion finale est apportée, après ingestion, par le liquide physiologique du milieu digestif.

2. Composition selon la revendication 1, caractérisée en ce que les glycérides polyglycolysés de la phase lipophile sont saturés et donnent une composition solide à température ambiante.

3. Composition selon la renvendication 1, caractérisée en ce que les glycérides polyglycolysés de la phase lipophile sont insaturés et donnent une composition liquide à température ambiante.

4. Composition selon l'une des revendications 1 à 3, caractérisée en ce que le mélange de glycérides polyglycolysés présente une HLB égale à 14.

5. Composition selon la revendication 3, caractérisée en ce que le mélange de glycérides polyglycolysés présente une HLB égale à 6 et est liquide à température ambiante.

6. Composition selon l'une des revendication 1 à 5, caractérisée en ce que la phase lipophile représente de 10 à 75 % du poids de la composition.

7. Composition selon l'une des revendications 1 à 6, caractérisé en ce que l'agent tensioactif (TA) est constitué par des esters oléiques du polyglycérol, de HLB égale à 10.

8. Composition selon l'une des revendications 1 à 7 caractérisée en ce que le rapport TA/CoTA est compris entre 1 et 2.

## Claims

1. Composition for pharmaceutical use intended to be ingested, able to form a microemulsion, comprising at least :
- an active ingredient,
- a lipophilic phase representing from 1 to 75 % of the total weight of the composition consisting of a mixture of glycerides and C8 to C18 fatty acid esters,
- a surfactant (SA), consisting of glycerides having a hydrophilic-lipophilic balance (HLB) of less than 16,
- a cosurfactant (CoSA), consisting of a polyvalent alcohol ester, the SA/CoSA ratio being between 0.5 and 6,
- a hydrophilic phase,
*characterized* :
- in that the glycerides of the lipophilic phase consist of a mixture of mono-, di- and triglycerides and polyethylene glycol mono- and di-esters, having a hydrophilic-lipophilic balance (HLB) of less than 16 ;
- in that the surfactant (SA) is chosen from the group comprising saturated C8-C10 polyglycolized glycerides and oleic esters of polyglycerol ;
- in that the cosurfactant (CoSA) is chosen from the group comprising lauric esters of propylene glycol, oleic esters of polyglycerol and ethyl diglycol ;
- and in that the hydrophilic phase of the final microemulsion is supplied, after ingestion, by the physiological liquid of the digestive medium.

2. Composition according to claim 1, characterized in that the polyglycolized glycerides of the lipophilic phase are saturated and give a composition which is solid at room temperature.

3. Composition according to claim 1, characterized in that the polyglycolized glycerides of the lipophilic phase are unsaturated and give a composition which is liquid at room temperature.

4. Composition according to one of claims 1 to 3, characterized in that the mixture of polyglycolized glycerides has an HLB equal to 14.

5. Composition according to claim 3, characterized in that the mixture of polyglycolized glycerides has an HLB equal to 6 and is liquid at room temperature.

6. Composition according to one of claims 1 to 5, characterized in that the lipophilic phase represents from 10 to 75 % of the weight of the composition.

7. Composition according to one of claims 1 to 6, characterized in that the surfactant (SA) consists of oleic esters of polyglycerol having an HLB equal to 10.

8. Composition according to one of claims 1 to 7, characterized in that the SA/CoSA ratio is between 1 and 2.

## Patentansprüche

1. Zur Einnahme bestimmte und zur Bildung einer Mikroemulsion geeignete Zusammensetzung zur pharmazeutischen Verwendung, die zumindest umfaßt:
. ein aktives Agens,
. eine lipophile Phase, die 1 bis 75 % des Gesamtgewichtes der Zusammensetzung ausmacht und aus einem Gemisch aus Glyceriden und C₈-C₁₈-Fettsäureestern besteht,
. ein Tensid (TA) auf Glyceridbasis mit einem hydrolipophilen Verhältnis (HLB), das kleiner als 16 ist,
. ein Co-Tensid (CoTA), das aus einem Ester polyvalenter Alkohole gebildet wird, wobei das Verhältnis TA/CoTA im Bereich von 0,5 bis 6 liegt,
. eine hydrophile Phase,
dadurch gekennzeichnet:
- daß die Glyceride der lipophilen Phase aus einem Gemisch aus Mono-, Di- und Triglyceriden und Mono- und Diestern des Polyethylenglykols mit einem hydro-lipophilen Verhältnis (HLB), das kleiner als 16 ist, bestehen;
- daß das Tensid (TA) ausgewählt ist aus der Gruppe bestehend aus gesättigten, polyglykolysierten Glyceriden mit C₈-C₁₀-Fettsäuren und Ölsäureestern des Polyglycerins;
- daß das Co-Tensid (CoTA) ausgewählt ist aus der Gruppe bestehend aus Laurinsäureestern des Propylenglykols, Ölsäureestern des Polyglycerins und Ethyldiglykol;
- und daß die hydrophile Phase der fertigen Mikroemulsion, nach der Einnahme, von der physiologischen Flüssigkeit des Verdauungsmilieus beigesteuert wird.

2. Zusammensetzung nach Anspruch 1, dadurch gekennzeichnet, daß die polyglykolysierten Glyceride der lipophilen Phase gesättigt sind und bei Raumtemperatur eine feste Zusammensetzung ergeben.

3. Zusammensetzung nach Anspruch 1, dadurch gekennzeichnet, daß die polyglykolysierten Glyceride der lipophilen Phase ungesättigt sind und bei Raumtemperatur eine flüssige Zusammensetzung ergeben.

4. Zusammensetzung nach einem der Ansprüche 1 bis 3, dadurch gekennzeichnet, daß das Gemisch aus polyglykolysierten Glyceriden einen HLB-Wert von 14 aufweist.

5. Zusammensetzung nach Anspruch 3, dadurch gekennzeichnet, daß das Gemisch aus polyglykolysierten Glyceriden einen HLB-Wert von 6 aufweist und bei Raumtemperatur flüssig ist.

6. Zusammensetzung nach einem der Ansprüche 1 bis 5, dadurch gekennzeichnet, daß die lipophile Phase 10 bis 75 Gew.-% der Zusammensetzung ausmacht.

7. Zusammensetzung nach einem der Ansprüche 1 bis 6, dadurch gekennzeichnet, daß das Tensid (TA) aus Ölsäureestern des Polyglycerins mit einem HLB-Wert von 10 aufgebaut ist.

8. Zusammensetzung nach einem der Ansprüche 1 bis 7, dadurch gekennzeichnet, daß das Verhältnis von TA/CoTA im Bereich von 1 bis 2 liegt.
